# EUROPEAN PATENT APPLICATION

(11) **EP 4 834 319 A1**
(43) Date of publication of application: **04.11.2026**
(21) Application number: 24912983.4
(22) Date of filing: 26.12.2024
(51) Int. Cl.: A61K 8/60, A61K 8/44, A61K 8/96, A61Q 19/00

(54) **TOPICAL PREPARATION FOR SKIN**

(30) Priority: 28.12.2023 JP 2023222698
(71) Applicant: Kao Corporation, Tokyo 103-8210 (JP)
(72) Inventor: SUMIDA, Yasuteru, Tokyo 103-8210 (JP)
(74) Representative: Hoffmann Eitle
(86) International application number: PCT/JP2024/046102
(87) International publication number: WO 2025/143097

(57) **Abstract**

The present invention provides a topical preparation for skin that does not generate odor or coloration even when stored at a high temperature, is excellent in storage stability, and does not feel sticky during or after application. The present invention relates to a topical preparation for skin comprising the following components (A), (B), and (C): (A) γ-amino-β-hydroxybutyric acid; (B) one or more selected from the group consisting of osmolytes; and (C) water.

## Description

### Field of the Invention

The present invention relates to a topical preparation for skin.

### Background of the Invention

γ-amino-β-hydroxybutyric acid has been used by being incorporated into cosmetics and the like.

For example, Patent Literature 1 states that a skin cosmetic containing γ-amino-β-hydroxybutyric acid is excellent in a skin anti-aging effect.

Patent Literature 2 states that a skin cosmetic containing γ-amino-β-hydroxybutyric acid and vitamin B₆ is excellent in a skin anti-aging effect and the sustainability of such an effect.

Patent Literature 3 states that a whitening cosmetic containing an extract obtained from Dioscorea composita and a medicinally effective ingredients such as γ-amino-β-hydroxybutyric acid prevents inflammation caused by ultraviolet rays, suppresses production of melanin due to ultraviolet damage, promotes excretion of melanin pigments, and exhibits an excellent whitening effect in a short period of time.

Patent Literature 1: JP-A-62-255405
Patent Literature 2: JP-A-63-10708
Patent Literature 3: JP-A-2003-246709

### Summary of the Invention

The present invention relates to a topical preparation for skin comprising the following components (A), (B), and (C):
(A) γ-amino-β-hydroxybutyric acid;
(B) one or two or more selected from the group consisting of osmolytes; and
(C) water.

### Detailed Description of the Invention

γ-amino-β-hydroxybutyric acid has had a problem, especially when water is present, of coloration and odor occurring over time during storage at a high temperature. In addition, it has also had a problem of stickiness felt upon and after application.

The present inventors have found that, by using the γ-amino-β-hydroxybutyric acid in combination with an osmolyte, a topical preparation for skin can be obtained, which does not generate odor or coloration even when stored at a high temperature, is excellent in storage stability, and does not feel sticky during or after application.

The topical preparation for skin according to the present invention does not generate odor or change in appearance even when stored at a high temperature, is excellent in storage stability, and does not feel sticky during or after application. Further, it also has no sliminess.

### Mode for Carrying Out the Invention

The component (A), that is, the γ-amino-β-hydroxybutyric acid, is a known substance and has the following chemical properties.
- Chemical name: γ-amino-β-hydroxybutyric acid
- Molecular formula: C₄H₉NO₃
- Molecular weight: 119.12
- Melting point: from 205 to 210°C
- Structural formula:

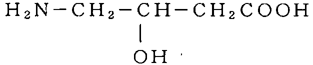

From the viewpoints of improving the skin anti-aging effect and reducing the stickiness during and after application, the content of the component (A) is preferably 0.001% by mass or more in the total composition, more preferably 0.005% by mass or more, further more preferably 0.05% by mass or more, even more preferably 0.1% by mass or more, and particularly preferably 0.3% by mass or more, and is preferably 3.5% by mass or less, more preferably 2% by mass or less, further more preferably 1.5% by mass or less, even more preferably 1.2% by mass or less, and particularly preferably 1% by mass or less. In addition, the content of the component (A) is preferably 0.001% by mass or more and 3.5% by mass or less in the total composition, more preferably 0.005% by mass or more and 2% by mass or less, further more preferably 0.05% by mass or more and 1.5% by mass or less, even more preferably 0.1% by mass or more and 1.2% by mass or less, and particularly preferably 0.3% by mass or more and 1% by mass or less.

The osmolyte of the component (B) is a chemical substance which mainly regulates an osmotic pressure in a living organism. In a cell, it has a function of maintaining a cell volume against the inflow or outflow of water caused by extracellular osmotic stress (osmotic pressure gradient), and also has a function of stabilizing the structure and function of proteins, such as enzymes, over a wide range of concentration and protecting the proteins from denaturation. It should be noted that the component (B) does not include a pH adjusting agent as component (D) which will be described later.

Examples of the osmolyte of the component (B) include ectoine, sodium pyrrolidone carboxylate (PCA-Na), arginine, arginine hydrochloride, carnitine chloride, taurine, glutamic acid, glutamate, serine, N-methyl-L-serine, trimethylglycine, alanine, and N-amidino-L-proline.

From the viewpoints of improving the stability during the storage at a high temperature, and reducing the stickiness during and after application, the osmolyte contained is preferably one or two or more selected from the group consisting of ectoine, sodium pyrrolidone carboxylate (PCA-Na), arginine, N-methyl-L-serine, trimethylglycine, alanine, and N-amidino-L-proline, more preferably one or two or more selected from the group consisting of ectoine, sodium pyrrolidone carboxylate (PCA-Na), arginine, N-methyl-L-serine, trimethylglycine, and N-amidino-L-proline, further more preferably one or two or more selected from the group consisting of ectoine, sodium pyrrolidone carboxylate (PCA-Na), arginine, trimethylglycine, and N-amidino-L-proline, even more preferably one or two or more selected from the group consisting of ectoine, sodium pyrrolidone carboxylate (PCA-Na), trimethylglycine, and N-amidino-L-proline, and is particularly preferably ectoine.

The component (B) may be used alone or in combination of two or more. From the viewpoints of improving the stability during the storage at a high temperature, and reducing the stickiness during and after application, the content of the component (B) is preferably 0.015% by mass or more in the total composition, more preferably 0.02% by mass or more, further more preferably 0.3% by mass or more, even more preferably 0.5% by mass or more, particularly preferably 0.8% by mass or more, and particularly preferably 0.9% by mass or more, and is preferably 3.5% by mass or less, more preferably 3% by mass or less, further more preferably 2.5% by mass or less, even more preferably 2% by mass or less, particularly preferably 1.5% by mass or less, and particularly preferably 1% by mass or less. In addition, the content of the component (B) is preferably 0.015% by mass or more and 3.5% by mass or less in the total composition, more preferably 0.02% by mass or more and 3% by mass or less, further more preferably 0.3% by mass or more and 2.5% by mass or less, even more preferably 0.5% by mass or more and 2% by mass or less, particularly preferably 0.8% by mass or more and 1.5% by mass or less, and particularly preferably 0.9% by mass or more and 1% by mass or less.

From the viewpoints of improving the stability during the storage at a high temperature, and reducing the stickiness during and after application, in the present invention, the molar ratio of the component (B) to the component (A), (B)/(A) is preferably 0.005 or more, more preferably 0.01 or more, further more preferably 0.1 or more, even more preferably 0.5 or more, and particularly preferably 0.75 or more, and is preferably 5 or less, more preferably 3 or less, further more preferably 2.5 or less, even more preferably 2 or less, and particularly preferably 0.85 or less. In addition, the molar ratio of the component (B) to the component (A), (B)/(A) is preferably 0.005 or more and 5 or less, more preferably 0.01 or more and 3 or less, further more preferably 0.1 or more and 2.5 or less, even more preferably 0.5 or more and 2 or less, and particularly preferably 0.75 or more and 0.85 or less.

From the viewpoints of uniformly dissolving the component (B) and reducing the stickiness during and after application, the content of water as the component (C) is preferably 20% by mass or more in the total composition, more preferably 40% by mass or more, and further more preferably 60% by mass or more, and is preferably 99.98% by mass or less, more preferably 90% by mass or less, and further more preferably 85% by mass or less. In addition, the content of water as the component (C) is preferably 20% by mass or more and 99.98% by mass or less in the total composition, more preferably 40% by mass or more and 90% by mass or less, and further more preferably 60% by mass or more and 85% by mass or less.

The topical preparation for skin according to the present invention may further contain a pH adjusting agent as component (D), and thus is capable of improving the stability during the storage at a high temperature. It should be noted that the component (D) does not include the osmolyte of the component (B).

Any pH adjusting agent may be used as long as it is commonly applicable for a cosmetic, and inorganic acids and salts thereof, organic acids having six or less carbon atoms and salts thereof are preferred, and organic acids having six or less carbon atoms and salts thereof are more preferred.

The inorganic acid contained is preferably one or two or more selected from the group consisting of, for example, hydrochloric acid, nitric acid, nitrous acid, sulfuric acid, sulfurous acid, phosphoric acid, phosphonic acid, phosphinic acid, and salts thereof, and is more preferably one or more selected from the group consisting of phosphoric acid and salts thereof.

As the organic acids having six or less carbon atoms and salts thereof, water-soluble organic acids and salts thereof are preferred, and examples thereof include fatty acids having from one to six carbon atoms, hydroxy acids, dicarboxylic acids, and salts thereof. Among these, the pH adjusting agent preferably contains one or two or more selected from the group consisting of malic acid, lactic acid, citric acid, succinic acid, acetic acid, tartaric acid, aspartic acid, adipic acid, and salts thereof, more preferably contains one or two or more selected from the group consisting of malic acid, lactic acid, citric acid, succinic acid, and salts thereof, and further more preferably contains one or more selected from the group consisting of succinic acid, citric acid and salts thereof.

The pH adjusting agent contained is preferably one or more selected from the group consisting of the organic acids having six or less carbon atoms and salts thereof, and more preferably one or more selected from the group consisting of succinic acid, citric acid, and salts thereof.

The component (D) may be used alone or in combination of two or more. From the viewpoint of improving the stability during the storage at a high temperature, the content of the component (D) is preferably 0.01% by mass or more in the total composition, more preferably 0.03% by mass or more, further more preferably 0.05% by mass or more, and even more preferably 0.08% by mass or more, and is preferably 1% by mass or less, more preferably 0.5% by mass or less, further more preferably 0.3% by mass or less, and even more preferably 0.1% by mass or less. In addition, the content of the component (D) is preferably 0.01% by mass or more and 1% by mass or less in the total composition, more preferably 0.03% by mass or more and 0.5% by mass or less, further more preferably 0.05% by mass or more and 0.3% by mass or less, and even more preferably 0.08% by mass or more and 0.1% by mass or less.

The topical preparation for skin according to the present invention may further contain a water-soluble polysaccharide.

The water-soluble polysaccharide is not particularly limited as long as it is commonly applicable for a cosmetic, and may be derived from a natural product or may be obtained by chemical synthesis.

In addition, the polysaccharide also includes polysaccharide derivatives obtained by bonding, to a part of the sugar moiety of a polysaccharide, an alkyl group, a hydroxyalkyl group, a polyalkylene oxide group such as a polyethylene oxide group and a polypropylene oxide group, a sugar such as glucose, lactose, and sucrose, or a macromolecule having such a sugar as a constitutional unit, as those equivalent to the polysaccharide.

Specific examples of the polysaccharide include locust bean gum, guar gum, tamarind gum, quince seed-derived gum, gum arabic, tragacanth gum, karaya gum, carrageenan, alginic acid, pectin, hydroxypropyl guar gum, methylcellulose, hydroxyethylcellulose, hydroxypropylcellulose, hydroxypropyl methylcellulose, carboxymethylcellulose, tuberose polysaccharide, xanthan gum, polysaccharides produced by Alcaligenes latus, gellan gum, dextran, pullulan, hyaluronic acid or a salt thereof, chondroitin sulfate, dermatan sulfate, and mucopolysaccharides such as chitin and chitosan.

Among these, from the viewpoint of improving moisturizing feel, it is preferred that the preparation contains one or two or more selected from the group consisting of carrageenan, hydroxypropyl methylcellulose, and hyaluronic acid or a salt thereof, and it is more preferred that the preparation contains at least hyaluronic acid or a salt thereof.

The water-soluble polysaccharide may be used alone or in combination of two or more. From the viewpoint of improving the feel when the topical preparation is applied, the content of the water-soluble polysaccharide is preferably 0.01% by mass or more in the total composition, more preferably 0.02% by mass or more, and further more preferably 0.03% by mass or more, and is preferably 1% by mass or less, more preferably 0.5% by mass or less, and further more preferably 0.1% by mass or less. In addition, the content of the water-soluble polysaccharide is preferably 0.01% by mass or more and 1% by mass or less in the total composition, more preferably 0.02% by mass or more and 0.5% by mass or less, and further more preferably 0.03% by mass or more and 0.1% by mass or less.

The topical preparation for skin according to the present invention may further contain a polyhydric alcohol. It should be noted that the polyhydric alcohol does not include the osmolyte of the component (B).

The polyhydric alcohol is a compound having two or more hydroxyl groups in the molecule, and any such compound may be used as long as it is commonly applicable for a topical preparation for skin.

Examples of dihydric alcohols include polyethylene glycol, propylene glycol, dipropylene glycol, tripropylene glycol, polypropylene glycol, 1,3-butylene glycol, and propanediol. Examples of trihydric alcohols include glycerin, and trimethylolpropane. Examples of tetrahydric alcohols include diglycerin, and erythritol. Examples of polyhydric alcohols having five or more hydroxyl groups include polyglycerins such as triglycerin; and sugars and sugar alcohols such as glucose, maltose, maltitol, sucrose, xylitol, sorbitol, malbitol, polyoxyethylene methyl glucoside, polyoxyethylene ethyl glucoside, and polyoxyethylene propylene glucoside.

From the viewpoint of improving the moisturizing feel, the polyhydric alcohol contained is preferably one or more selected from the group consisting of dihydric alcohols, trihydric alcohols, tetrahydric alcohols, sugars, and sugar alcohols, more preferably one or two or more selected from the group consisting of trihydric alcohols, tetrahydric alcohols, sugars, and sugar alcohols, and further more preferably one or two or more selected from the group consisting of glycerin, diglycerin, sorbitol, and glucose.

The polyhydric alcohol may be used alone or in combination of two or more. From the viewpoint of improving the feel when the topical preparation is applied, the content of the polyhydric alcohol is preferably 1% by mass or more in the total composition, more preferably 3% by mass or more, and further more preferably 5% by mass or more, and is preferably 20% by mass or less, more preferably 15% by mass or less, and further more preferably 12% by mass or less. In addition, the content of the polyhydric alcohol is preferably 1% by mass or more and 20% by mass or less in the total composition, more preferably 3% by mass or more and 15% by mass or less, and further more preferably 5% by mass or more and 12% by mass or less.

In addition to the components described above, the topical preparation for skin according to the present invention may contain components commonly applicable for a topical preparation for skin, such as, oil-based components, surfactants, preservatives, antioxidants, colorants, fragrances, plant extracts, coloring agents, powders, ultraviolet absorbing agents, moisturizing agents other than the polyhydric alcohol, blood circulation promoting agents, cooling agents, antiperspirant agents, bactericides, and skin activating agents.

When diisopropylamidinomonochloroacetic acid is contained, from the viewpoints of improving the stability during the storage at a high temperature, and reducing the stickiness during and after application, the content thereof is preferably 0.1% by mass or less, more preferably 0.05% by mass or less, and further more preferably 0.01% by mass or less. It is even more preferred that it be substantially free of diisopropylamidinomonochloroacetic acid.

The topical preparation for skin according to the present invention can also be used as an agent for alleviating hypersensitivity.

The topical preparation for skin according to the present invention can be produced according to a conventional method.

For example, it can be produced by adding the component (A), the component (B), and other components while stirring water, and then stirring the mixture.

From the viewpoint of enhancing transdermal absorbability, the topical preparation for skin according to the present invention preferably has a pH value of from 4 to 7, more preferably from 4.5 to 6.5, and further more preferably from 5 to 6.

In the present invention, the pH value is measured at 25°C using a bench-top pH meter F-72 manufactured by HORIBA, Ltd.

The topical preparation for skin according to the present invention can be applied to cosmetics, quasi-drug medicated cosmetics, pharmaceuticals, and the like. It is suitable as cosmetics, more preferably as cosmetics for skin care, such as a lotion for skin care, an emulsion for skin care, a cream for skin care, a BB cream, and a beauty serum, and further more preferably as a lotion for skin care.

Regarding the embodiments described above, the present invention further discloses the following compositions.
<1> A topical preparation for skin comprising the following components (A), (B), and (C):
   (A) γ-amino-β-hydroxybutyric acid;
   (B) one or two or more selected from the group consisting of osmolytes; and
   (C) water.
<2> The topical preparation for skin according to <1>, wherein a content of the component (A) is preferably 0.001% by mass or more and 3.5% by mass or less in the total composition, more preferably 0.005% by mass or more and 2% by mass or less, further more preferably 0.05% by mass or more and 1.5% by mass or less, even more preferably 0.1% by mass or more and 1.2% by mass or less, and particularly preferably 0.3% by mass or more and 1% by mass or less.
<3> The topical preparation for skin according to <1> or <2>, wherein the component (B) preferably contains one or two or more selected from the group consisting of ectoine, sodium pyrrolidone carboxylate (PCA-Na), arginine, N-methyl-L-serine, trimethylglycine, alanine, and N-amidino-L-proline, more preferably contains one or two or more selected from the group consisting of ectoine, sodium pyrrolidone carboxylate (PCA-Na), arginine, N-methyl-L-serine, trimethylglycine, and N-amidino-L-proline, further more preferably contains one or two or more selected from the group consisting of ectoine, sodium pyrrolidone carboxylate (PCA-Na), arginine, trimethylglycine, and N-amidino-L-proline, even more preferably contains one or two or more selected from the group consisting of ectoine, sodium pyrrolidone carboxylate (PCA-Na), trimethylglycine, and N-amidino-L-proline, and particularly preferably contains ectoine.
<4> The topical preparation for skin according to any one of <1> to <3>, wherein a content of the component (B) is preferably 0.015% by mass or more and 3.5% by mass or less in the total composition, more preferably 0.02% by mass or more and 3% by mass or less, further more preferably 0.3% by mass or more and 2.5% by mass or less, even more preferably 0.5% by mass or more and 2% by mass or less, particularly preferably 0.8% by mass or more and 1.5% by mass or less, and particularly preferably 0.9% by mass or more and 1% by mass or less.
<5> The topical preparation for skin according to any one of <1> to <4>, wherein a molar ratio of the component (B) to the component (A), (B)/(A) is preferably 0.005 or more and 5 or less, more preferably 0.01 or more and 3 or less, further more preferably 0.1 or more and 2.5 or less, even more preferably 0.5 or more and 2 or less, and particularly preferably 0.75 or more and 0.85 or less.
<6> The topical preparation for skin according to any one of <1> to <5>, wherein a content of the component (C) is preferably 20% by mass or more and 99.98% by mass or less in the total composition, more preferably 40% by mass or more and 90% by mass or less, and further more preferably 60% by mass or more and 85% by mass or less.
<7> A topical preparation for skin comprising the following components (A), (B), and (C):
   (A) γ-amino-β-hydroxybutyric acid at a content of 0.3% by mass or more and 3.5% by mass or less;
   (B) one or two or more selected from the group consisting of osmolytes; and
   (C) water.
<8> A topical preparation for skin comprising the following components (A), (B), and (C):
   (A) γ-amino-β-hydroxybutyric acid at a content of 0.3% by mass or more and 3.5% by mass or less;
   (B) one or two or more selected from the group consisting of ectoine, sodium pyrrolidone carboxylate, arginine, trimethylglycine, N-methyl-L-serine, and N-amidino-L-proline; and
   (C) water.
<9> The topical preparation for skin according to <7> or <8>, wherein a content of the component (B) is 0.015% by mass or more and 3.5% by mass or less in the total composition.
<10> A topical preparation for skin comprising the following components (A), (B), and (C):
   (A) γ-amino-β-hydroxybutyric acid;
   (B) one or two or more selected from the group consisting of ectoine, sodium pyrrolidone carboxylate, arginine, trimethylglycine, and N-amidino-L-proline; and
   (C) water.
<11> A topical preparation for skin comprising the following components (A), (B), and (C):
   (A) γ-amino-β-hydroxybutyric acid;
   (B) ectoine; and
   (C) water.
<12> The topical preparation for skin according to <10> or <11>, wherein a content of the component (A) is 0.001% by mass or more and 3.5% by mass or less in the total composition and a content of the component (B) is 0.015% by mass or more and 3.5% by mass or less in the total composition.
<13> The topical preparation for skin according to any one of <1> to <10>, further comprising one or more selected from the group consisting of a pH adjusting agent as component (D), a water-soluble polysaccharide, and a polyhydric alcohol.

### Examples

### Examples 1 to 20 and Comparative Example 1

Topical preparations for skin (lotions) having the compositions shown in Tables 1 and 2 were produced, and the pH value was measured, and the odor after storage at 50°C for one month, the appearance after storage at 50°C for one month, as well as the non-stickiness during and after application were evaluated. The results are also shown in Table 1. In Table 1, PCA-Na indicates sodium pyrrolidone carboxylate.

### (Preparation Method)

At 25°C, the component (A), the component (B), and other components were added while stirring water, and the mixture was then stirred to prepare a topical preparation for skin (lotion).

### (Evaluation Method)

### (1) pH value:

For each topical preparation for skin immediately after the preparation, the pH value was measured at 25°C using a bench-top pH meter F-72 manufactured by HORIBA, Ltd.

### (2) Odor after storage at 50°C for one month:

A 50 mL glass bottle was filled with 40 g of each topical preparation for skin, and stored in a constant-temperature chamber at 50°C for one month. After the storage, the glass bottle containing the topical preparation for skin was taken out from the constant-temperature chamber at 50°C and allowed to stand still at 25°C for six hours. Thereafter, the lid of the glass bottle was opened, and the odor of the topical preparation for skin was evaluated by sensory evaluation according to the following criteria.
A: Only the original odor of the base material is perceived.
B: A weak off-odor is perceived.
C: A distinct off-odor is perceived.

### (3) Appearance after storage at 50°C for one month:

A 50 mL glass bottle was filled with 40 g of each topical preparation for skin, and stored in a constant-temperature chamber at 50°C for one month. After the storage, the glass bottle containing the topical preparation for skin was taken out from the constant-temperature chamber at 50°C and allowed to stand still at 25°C for six hours. Thereafter, the appearance of the cosmetic was observed with the naked eye and evaluated according to the following criteria.
A: It is colorless and transparent.
B: Slight coloration is observed.
C: Distinct coloration is observed.

### (4) Non-stickiness during and after application:

Approximately 0.2 g of each skin cosmetic was applied to the back of the hand by ten expert panelists using their fingers. The non-stickiness during and after application was evaluated according to the following criteria. The results are shown as the total score of the ten panelists.
3 points: No stickiness is felt.
2 points: Slight stickiness is felt.
1 point: Strong stickiness is felt.

**[Table 1]**

| Component (% by mass) | | Examples | | | | | | | | | | | | Comparative Example |
|---|---|---|---|---|---|---|---|---|---|---|---|---|---|---|
| | | 1 | 2 | 3 | 4 | 5 | 6 | 7 | 8 | 9 | 10 | 11 | 12 | 1 |
| (A) | γ-amino-β-hydroxybutyric acid | 1 | 1 | 1 | 1 | 1 | 1 | 1 | 1 | 1 | 1 | 1 | 1 | 1 |
| (B) | Ectoine | 0.02 | 1 | 3 | | | | | | | | | | |
| | Trimethylglycine | | | | 0.02 | 1 | 3 | | | | | | | |
| | PCA-Na | | | | | | | 0.02 | 1 | 3 | | | | |
| | N-methyl-L-serine | | | | | | | | | | 0.02 | 1 | 2 | |
| (D) | Citric acid | 0.02 | 0.02 | 0.02 | 0.02 | 0.02 | 0.02 | 0.02 | 0.02 | 0.02 | 0.02 | 0.02 | 0.02 | 0.02 |
| | Succinic acid | 0.02 | 0.02 | 0.02 | 0.02 | 0.02 | 0.02 | 0.02 | 0.02 | 0.02 | 0.02 | 0.02 | 0.02 | 0.02 |
| | Trisodium citrate | 0.06 | 0.06 | 0.06 | 0.06 | 0.06 | 0.06 | 0.06 | 0.06 | 0.06 | 0.06 | 0.06 | 0.06 | 0.06 |
| | Glycerin | 5 | 5 | 5 | 5 | 5 | 5 | 5 | 5 | 5 | 5 | 5 | 5 | 5 |
| | Diglycerin | 3 | 3 | 3 | 3 | 3 | 3 | 3 | 3 | 3 | 3 | 3 | 3 | 3 |
| | Sorbitol | 3 | 3 | 3 | 3 | 3 | 3 | 3 | 3 | 3 | 3 | 3 | 3 | 3 |
| | Glucose | 1 | 1 | 1 | 1 | 1 | 1 | 1 | 1 | 1 | 1 | 1 | 1 | 1 |
| | Sodium hyaluronate (from 10,000 to 100,000) | 0.05 | 0.05 | 0.05 | 0.05 | 0.05 | 0.05 | 0.05 | 0.05 | 0.05 | 0.05 | 0.05 | 0.05 | 0.05 |
| | Sodium hyaluronate (from 1,000,000 to 3,000,000) | 0.01 | 0.01 | 0.01 | 0.01 | 0.01 | 0.01 | 0.01 | 0.01 | 0.01 | 0.01 | 0.01 | 0.01 | 0.01 |
| | Phytic acid | 0.05 | 0.05 | 0.05 | 0.05 | 0.05 | 0.05 | 0.05 | 0.05 | 0.05 | 0.05 | 0.05 | 0.05 | 0.05 |
| | Sodium benzoate | 0.2 | 0.2 | 0.2 | 0.2 | 0.2 | 0.2 | 0.2 | 0.2 | 0.2 | 0.2 | 0.2 | 0.2 | 0.2 |
| (C) | Water | Balance | Balance | Balance | Balance | Balance | Balance | Balance | Balance | Balance | Balance | Balance | Balance | Balance |
| Total | | 100 | 100 | 100 | 100 | 100 | 100 | 100 | 100 | 100 | 100 | 100 | 100 | 100 |
| (A) Total amount | | 1 | 1 | 1 | 1 | 1 | 1 | 1 | 1 | 1 | 1 | 1 | 1 | 1 |
| (B) Total amount | | 0.02 | 1 | 3 | 0.02 | 1 | 3 | 0.02 | 1 | 3 | 0.02 | 1 | 2 | 0 |
| (D) Total amount | | 0.1 | 0.1 | 0.1 | 0.1 | 0.1 | 0.1 | 0.1 | 0.1 | 0.1 | 0.1 | 0.1 | 0.1 | 0.1 |
| (E) Total amount | | 0.06 | 0.06 | 0.06 | 0.06 | 0.06 | 0.06 | 0.06 | 0.06 | 0.06 | 0.06 | 0.06 | 0.06 | 0.06 |
| Molar ratio (B)/(A) | | 0.016 | 0.8 | 2.4 | 0.018 | 0.9 | 2.7 | 0.014 | 0.7 | 2.1 | 0.018 | 0.9 | 2.7 | - |
| pH | | 5.5 | 5.5 | 5.5 | 5.5 | 5.5 | 5.5 | 5.5 | 5.5 | 5.5 | 5.5 | 5.5 | 5.5 | 5.5 |
| Odor after storage at 50°C for one month | | A | A | A | A | A | A | A | A | A | A | A | A | C |
| Appearance after storage at 50°C for one month | | A | A | A | A | A | A | A | A | A | A | A | A | C |
| Non-stickiness during and after application | | 23 | 27 | 20 | 24 | 24 | 21 | 24 | 23 | 20 | 22 | 25 | 21 | 25 |

**[Table 2]**

| Component (% by mass) | | Examples | | | | | | | |
|---|---|---|---|---|---|---|---|---|---|
| | | 13 | 14 | 15 | 16 | 17 | 18 | 19 | 20 |
| (A) | γ-amino-β-hydroxybutyric acid | 1 | 1 | 1 | 0.1 | 2 | 1 | 1 | 1 |
| (B) | Ectoine | | | | 0.3 | 1 | | | |
| | Arginine | 0.02 | 1 | 2 | | | | | |
| | N-amidino-L-proline | | | | | | 0.02 | 1 | 2 |
| (D) | Citric acid | 0.03 | 0.3 | 0.3 | 0.02 | 0.02 | 0.02 | 0.02 | 0.02 |
| | Succinic acid | 0.03 | 0.3 | 0.7 | 0.02 | 0.02 | 0.02 | 0.02 | 0.02 |
| | Trisodium citrate | 0.04 | - | - | 0.06 | 0.06 | 0.06 | 0.06 | 0.06 |
| | Glycerin | 5 | 5 | 5 | 5 | 5 | 5 | 5 | 5 |
| | Diglycerin | 3 | 3 | 3 | 3 | 3 | 3 | 3 | 3 |
| | Sorbitol | 3 | 3 | 3 | 3 | 3 | 3 | 3 | 3 |
| | Glucose | 1 | 1 | 1 | 1 | 1 | 1 | 1 | 1 |
| | Sodium hyaluronate (from 10,000 to 100,000) | 0.05 | 0.05 | 0.05 | 0.05 | 0.05 | 0.05 | 0.05 | 0.05 |
| | Sodium hyaluronate (from 1,000,000 to 3,000,000) | 0.01 | 0.01 | 0.01 | 0.01 | 0.01 | 0.01 | 0.01 | 0.01 |
| | Phytic acid | 0.05 | 0.05 | 0.05 | 0.05 | 0.05 | 0.05 | 0.05 | 0.05 |
| | Sodium benzoate | 0.2 | 0.2 | 0.2 | 0.2 | 0.2 | 0.2 | 0.2 | 0.2 |
| (C) | Water | Balance | Balance | Balance | Balance | Balance | Balance | Balance | Balance |
| Total | | 100 | 100 | 100 | 100 | 100 | 100 | 100 | 100 |
| (A) Total amount | | 1 | 1 | 1 | 0.1 | 2 | 1 | 1 | 1 |
| (B) Total amount | | 0.02 | 1 | 2 | 0.3 | 1 | 0.02 | 1 | 2 |
| (D) Total amount | | 0.1 | 0.6 | 1 | 0.1 | 0.1 | 0.1 | 0.1 | 0.1 |
| (E) Total amount | | 0.06 | 0.06 | 0.06 | 0.06 | 0.06 | 0.06 | 0.06 | 0.06 |
| Molar ratio (B)/(A) | | 0.014 | 0.7 | 1.4 | 2.5 | 0.4 | 0.013 | 0.7 | 2.1 |
| pH | | 5.5 | 6 | 6.5 | 5.5 | 5.5 | 5.5 | 5.5 | 5.5 |
| Odor after storage at 50°C for one month | | A | A | A | A | A | A | A | A |
| Appearance after storage at 50°C for one month | | A | A | A | A | A | A | A | A |
| Non-stickiness during and after application | | 24 | 22 | 20 | 25 | 24 | 22 | 24 | 21 |

### Formulation Example 1 (Lotion)

### (Components)

| | | |
|---|---|---|
| 1. | γ-amino-β-hydroxybutyric acid: | 0.3 (% by mass) |
| 2. | Allantoin: | 0.5 |
| 3. | Citric acid: | appropriate amount (from 0.01 to 0.2) |
| 4. | Succinic acid: | appropriate amount (from 0.01 to 0.2) |
| 5. | Trisodium citrate: | appropriate amount (from 0.01 to 0.2) |
| 6. | Glycerin: | 5.0 |
| 7. | Diglycerin: | 2.0 |
| 8. | Sodium hyaluronate: | 0.1 |
| 9. | Carrageenan: | 0.15 |
| 10. | Ectoine: | 0.7 |
| 11. | N-methyl-L-serine: | 0.1 |
| 12. | Sodium benzoate: | 0.2 |
| 13. | Water: | balance |
| Total: 100 | | |

### (Preparation Method)

While stirring water, the components 1 to 12 are sequentially added and dissolved, followed by filtration to prepare a lotion (with a pH value of from 4 to 7).

### Formulation Example 2 (Beauty Serum)

### (Components)

| | | |
|---|---|---|
| 1. | Ascorbic acid 2-glucoside: | 3.0 (% by mass) |
| 2. | γ-amino-β-hydroxybutyric acid: | 1.5 |
| 3. | Citric acid: | appropriate amount (from 0.01 to 0.2) |
| 4. | Trisodium citrate: | appropriate amount (from 0.01 to 0.2) |
| 5. | Potassium hydroxide: | appropriate amount (from 0.1 to 1.0) |
| 6. | Glycerin: | 3.0 |
| 7. | Diglycerin: | 1.0 |
| 8. | 1,3-Butylene glycol: | 5.0 |
| 9. | Carboxyvinyl polymer: | appropriate amount (from 0.1 to 1.0) |
| 10. | Sodium hyaluronate: | 0.05 |
| 11. | Alanine: | 0.5 |
| 12. | Ectoine: | 0.05 |
| 13. | Arginine: | appropriate amount (from 0.1 to 1.0) |
| 14. | Phenoxyethanol: | 0.3 |
| 15. | Polyoxyethylene hydrogenated castor oil (60 E.O.): | 0.2 |
| 16. | Fragrance: | appropriate amount |
| 17. | Water: | balance |
| Total: 100 | | |

### (Preparation Method)

While stirring water, the components 1 to 13 are sequentially added and dissolved. Separately, the components 15 and 16 are mixed and then added to the previously filtered aqueous phase to prepare a beauty serum (with a pH value of from 6 to 7).

The amount of the component 9 blended is adjusted so that the final viscosity of the beauty serum measured at 30°C using a B-type viscometer is from 1,000 to 20,000 mPa·s.

### Formulation Example 3 (Emulsion)

### (Components)

| | | |
|---|---|---|
| 1. | γ-amino-β-hydroxybutyric acid: | 0.2 (% by mass) |
| 2. | Nicotinamide: | 1.0 |
| 3. | Citric acid: | appropriate amount (from 0.01 to 0.2) |
| 4. | Trisodium citrate: | appropriate amount (from 0.01 to 0.2) |
| 5. | Glycerin: | 5.0 |
| 6. | Carrageenan: | 0.2 |
| 7. | N-Methyl-L-serine: | 0.2 |
| 8. | Trimethylglycine: | 0.3 |
| 9. | Water: | balance |
| 10. | Hydrogenated soybean phospholipid (with a PC content | |
| | offrom 50 to 95%): | 0.5 |
| 11. | Cholesterol (phytosterol): | 0.5 |
| 12. | Sugar squalane: | 8.0 |
| 13. | Polyglyceryl-10 diisostearate: | 1.0 |
| 14. | Octyldodecyl myristate: | 3.0 |
| 15. | Isostearic acid: | 0.5 |
| 16. | Palmitic acid: | 0.1 |
| 17. | Dipropylene glycol: | 5.0 |
| 18. | 1,3-Butylene glycol: | 3.0 |
| 19. | Fragrance: | appropriate amount (from 0 to 0.5) |
| 20. | Phenoxyethanol: | 0.5 |
| 21. | Carboxyvinyl polymer: | appropriate amount (from 0.1 to 1.0) |
| 22. | Potassium hydroxide: | appropriate amount |
| Total: 100 | | |

### (Preparation Method)

The components 1 to 9 are stirred and dissolved at 80°C, and gradually added to the components 10 to 19 which were previously dissolved at 80°C, followed by dispersion and emulsification using a homomixer (HM), and then cooling. At around 50°C, the components 20 to 22 are sequentially added and dispersed again using the HM, followed by cooling to 30°C to prepare an emulsion (with a pH value of from 5 to 7).

The amount of the component 21 blended is adjusted so that the final viscosity of the emulsion measured at 30°C using a B-type viscometer is from 1,500 to 20,000 mPa·s.

### Formulation Example 4 (Cream)

### (Components)

| | | |
|---|---|---|
| 1. | γ-amino-β-hydroxybutyric acid: | 2.0 (% by mass) |
| 2. | Citric acid: | appropriate amount (from 0.01 to 0.2) |
| 3. | Trisodium citrate: | appropriate amount (from 0.01 to 0.2) |
| 4. | Phytic acid: | 0.1 |
| 5. | Sodium benzoate: | 0.2 |
| 6. | Dipotassium glycyrrhizate: | 0.1 |
| 7. | Glycerin: | 5.0 |
| 8. | Maltitol: | 8.0 |
| 9. | Sodium hyaluronate: | 0.1 |
| 10. | PCA-Na: | 1.0 |
| 11. | Ectoine: | 0.5 |
| 12. | Water: | balance |
| 13. | Hydrogenated soybean phospholipid: | 1.0 |
| 14. | Cholesterol (phytosterol): | 0.5 |
| 15. | Palmitic acid: | 0.2 |
| 16. | Behenyl alcohol: | 1.0 |
| 17. | Vegetable squalane: | 10.0 |
| 18. | Isostearic acid: | 0.5 |
| 19. | Cetyl alcohol: | 0.5 |
| 20. | Macadamia nut oil: | 3.0 |
| 21. | Jojoba oil: | 3.0 |
| 22. | Dipropylene glycol: | 5.0 |
| 23. | 1,3-Butylene glycol: | 3.0 |
| 24. | Fragrance: | appropriate amount (from 0 to 0.5) |
| 25. | Carboxyvinyl polymer: | appropriate amount (from 0.1 to 1.0) |
| 26. | Potassium hydroxide: | appropriate amount |
| Tot al: 100 | | |

### (Preparation Method)

The components 1 to 12 are stirred and dissolved at 80°C, and gradually added to the components 13 to 24 which were previously dissolved at 80°C, followed by dispersion and emulsification using a homomixer (HM), and then cooling. At around 50°C, the components 25 and 26 are sequentially added and dispersed again using the HM, followed by cooling to 30°C to prepare a cream (with a pH value of from 5 to 7).

The amount of the component 25 blended is adjusted so that the final viscosity of the cream measured at 30°C using a B-type viscometer is from 10,000 to 300,000 mPa·s.

Any of the topical preparations for skin according to the Formulation Examples 1 to 4 does not generate odor or coloration even when stored at a high temperature, is excellent in storage stability, and does not feel sticky during or after application.

## Claims

1. A topical preparation for skin comprising the following components (A), (B), and (C):
(A) γ-amino-β-hydroxybutyric acid;
(B) one or two or more selected from the group consisting of osmolytes; and
(C) water.

2. The topical preparation for skin according to claim 1, wherein the component (B) is one or two or more selected from the group consisting of ectoine, sodium pyrrolidone carboxylate, arginine, arginine hydrochloride, carnitine chloride, taurine, glutamic acid, glutamate, serine, N-methyl-L-serine, trimethylglycine, and alanine.

3. The topical preparation for skin according to claim 1 or 2, wherein a molar ratio of the component (B) to the component (A), (B)/(A) is from 0.005 to 5.

4. The topical preparation for skin according to any one of claims 1 to 3, wherein a content of the component (A) is from 0.001 to 3.5% by mass.

5. The topical preparation for skin according to any one of claims 1 to 4, wherein a content of the component (B) is from 0.015 to 3.5% by mass.

6. The topical preparation for skin according to any one of claims 1 to 5, further comprising a pH adjusting agent as component (D).
